# FASCICULE DE BREVET EUROPEEN

(11) **EP 0 889 871 B1**
(45) Date de publication et mention de la délivrance du brevet: **31.10.2001**
(21) Numéro de dépôt: 97919428.9
(22) Date de dépôt: 27.03.1997
(51) Int. Cl.: C07C 53/08, C07C 51/353, C07C 69/14, C07C 67/293

(54) **PROCEDE DE PREPARATION D'ACIDE ACETIQUE ET/OU D'ACETATE DE METHYLE PAR ISOMERISATION ET CARBONYLATION**
VERFAHREN ZUR HERSTELLUNG VON ESSIGSÄURE UND/ODER VON METHYLAZETAT DURCH ISOMERISIERUNG UND KARBONYLIERUNG
METHOD FOR PREPARING ACETIC ACID AND/OR METHYL ACETATE BY ISOMERISATION AND CARBONYLATION

(30) Priorité: 27.03.1996 FR 9603781; 10.07.1996 FR 9608590
(43) Date de publication de la demande: 13.01.1999
(73) Titulaire: ACETEX CHIMIE, 92205 Neuilly sur Seine Cedex (FR)
(72) Inventeur: PATOIS, Carl, F-69003 Lyon (FR); PERRON, Robert, F-69390 Charly (FR); THIEBAUT, Daniel, F-64140 Billère (FR)
(74) Mandataire: Giraud, Françoise
(86) Numéro de dépôt international: FR9700551
(87) Numéro de publication internationale: WO9735828

(56) Documents cités:
- EP-A- 0 045 637
- DE-A- 3 046 899
- US-A- 4 613 693

## Description

La présente invention a pour objet la préparation d'acide acétique et/ou d'acétate de méthyle, en mettant en oeuvre une réaction d'isomérisation et une réaction de carbonylation.

Diverses voies d'accès à l'acide acétique sont connues et exploitées industriellement. Parmi celles-ci figure la réaction de carbonylation du méthanol. Cette réaction de carbonylation peut notamment être mise en oeuvre en phase liquide, sous pression de monoxyde de carbone, qui est l'un des réactifs, en présence d'un système catalytique homogène comprenant un composé à base de rhodium et/ou d'iridium et un promoteur iodé.

Une autre voie d'accès est constituée par la réaction d'isomérisation de formiate de méthyle en présence d'un catalyseur à base de rhodium ou d'iridium.

Les procédés connus d'isomérisation catalysée avec de l'iridium, sont mis en oeuvre sous une atmosphère d'azote. Il a en effet été constaté que le monoxyde de carbone durant cette réaction, n'apportait pas d'avantage particulier et pouvait même être la cause d'une certaine inhibition de la réaction d'isomérisation, favorisant les réactions secondaires. Il est à noter qu'un tel comportement est tout à fait différent de celui observé lorsque le système catalytique est à base de rhodium, cas pour lequel la présence de monoxyde de carbone est essentielle au maintien en phase homogène du métal. Ce type de procédé, catalysé à l'iridium, dont l'intérêt n'est pas remis ici en cause ne présente toutefois pas un véritable intérêt sur le plan industriel car la réaction qui y est décrite n'est pas suffisamment performante. En effet, les vitesses de réaction sont seulement de l'ordre de 2 mol/h/l d'acide et/ou d'ester produits.

Il a été proposé, afin d'améliorer les résultats du procédé précité, de mettre en oeuvre la réaction d'isomérisation en présence d'un acide fort du type des acides sulfoniques, comme l'acide paratoluène sulfonique par exemple. Dans les conditions de ce procédé, la réaction est effectuée en mettant en oeuvre des quantités importantes de formiate de méthyle à isomériser, qui est par conséquent aussi utilisé comme solvant de la réaction. Si ce perfectionnement contribue à augmenter l'activité de la réaction, il présente cependant l'inconvénient de nécessiter l'emploi d'un composé supplémentaire, ce qui ne simplifie pas le procédé.

En outre, il est possible que cet acide se dégrade dans les conditions de composition du milieu réactionnel et de son utilisation.

La présente invention a pour but de proposer un procédé de préparation d'acide acétique et/ou d'acétate de méthyle en mettant en oeuvre simultanément une réaction d'isomérisation de formiate de méthyle et une réaction de carbonylation d'un réactif apportant un radical méthyle, tel que par exemple le méthanol.

Un tel procédé présente l'avantage d'assouplir les conditions de fonctionnement du procédé d'isomérisation ou même de celui de carbonylation.

En effet, il est possible d'utiliser du formiate de méthyle commercial, c'est-à-dire comprenant du méthanol à hauteur de quelques pour-cent, et l'on constate alors que la présence de cet alcool apporte sa contribution à la production d'acide acétique et/ou de l'ester correspondant. Par ailleurs, la possibilité de mettre en oeuvre l'isomérisation du formiate de méthyle peut permettre d'augmenter la capacité de production d'une installation de carbonylation du méthanol, sans avoir à engager des investissements importants nécessaires pour augmenter la production de monoxyde de carbone qui représente l'un des réactifs employés pour cette réaction.

En outre, et que ce soit dans l'un ou l'autre cas ci-dessus, apporter une diversification des réactifs est un avantage bien compréhensible.

Il est tout à fait remarquable que, malgré des conditions de mises en oeuvre qui ne lui sont a priori pas favorables, la réaction de carbonylation ait lieu, avec une bonne productivité.

Ces buts et d'autres sont atteints par la présente invention qui a donc pour objet un procédé de préparation d'acide acétique et/ou d'acétate de méthyle par réaction de réactifs apportant des radicaux formyles et de réactifs apportant des radicaux méthyles, en présence de monoxyde de carbone, d'eau, d'un solvant et d'un système catalytique comprenant au moins un promoteur halogéné et au moins un composé à base d'iridium, caractérisé en ce que ledit procédé est un procédé continu dans lequel on maintient une pression partielle en monoxyde de carbone comprise entre 0,1.10⁵ Pa et 25.10⁵ Pa, une quantité en réactif apportant les radicaux formyles inférieure ou égale à 20 % en poids du mélange réactionnel, on alimente les réactifs apportant des radicaux méthyles et formyles dans un rapport molaire des radicaux méthyles aux radicaux formyles supérieur à 1 et on maintient une quantité d'eau comprise entre 0 exclu et 5 % en poids du mélange réactionnel.

Les conditions ci-dessus permettent de réaliser simultanément l'isomérisation de l'ester issu des radicaux formyles et la carbonylation des radicaux méthyles alimentés.

On a en effet constaté que, contrairement à ce qui était prétendu dans l'art antérieur, la présence de monoxyde de carbone était essentielle pour la réaction d'isomérisation de l'ester en présence d'iridium.

Par ailleurs, le monoxyde de carbone est consommé et intervient en tant que réactif dans le réacteur de carbonylation.

Selon la seconde caractéristique ci-dessus, la teneur en réactif apportant les radicaux formyles est maintenue inférieure à 20 % en poids du mélange réactionnel.

Par ailleurs, le rapport des radicaux méthyles aux radicaux formyles permet de déterminer la nature de la ou des réactions qui sont mises en oeuvre durant le procédé. En effet, lorsque ce rapport est égal à 1, seule la réaction d'isomérisation est mise en oeuvre et cela sort du cadre de la présente invention. Tandis que si ce rapport est supérieur à 1, les deux réactions d'isomérisation et de carbonylation ont lieu.

Pour plus de clarté, le système catalytique va tout d'abord être décrit.

Le procédé de l'invention est donc mis en oeuvre en présence d'un système catalytique comprenant au moins un promoteur halogéné et au moins un composé à base d'iridium.

Le promoteur halogéné, représentant l'un des constituants du système catalytique, est choisi de préférence parmi les composés iodés.

Le promoteur halogéné peut se présenter sous la forme d'iode seul, ou en combinaison avec d'autres éléments comme par exemple l'hydrogène, un radical alkyle en C1-C10, un radical acyle en C1-C10, ou encore des iodures de métal alcalin ou des iodures métalliques, tels que les iodures de métaux de transition, ou de la colonne IIA de la classification périodique des éléments.

Il est à noter que le promoteur halogéné peut être constitué d'un mélange de plusieurs des promoteurs précités.

On ne sortirait pas du cadre de la présente invention en préparant in situ lesdits promoteurs halogénés, à l'aide de précurseurs appropriés.

Selon un mode de réalisation particulièrement avantageux de l'invention, le promoteur halogéné est choisi parmi l'iode, l'acide iodhydrique, l'iodure de méthyle, l'iodure d'aluminium, l'iodure de chrome, l'iodure de lithium, l'iodure de potassium ; ces composés étant utilisés seuls ou en mélange. De préférence, le promoteur halogéné comprend l'iode et un radical du type méthyle.

Par ailleurs, la quantité de promoteur halogéné maintenue pendant la réaction est plus particulièrement comprise entre 0,1 et 20 % en poids du mélange réactionnel. De préférence, la teneur en promoteur halogéné est comprise entre 1 et 15 % en poids du mélange réactionnel.

Il est à noter que les quantités de promoteur indiquées ci-dessus sont données à titre indicatif. En effet, l'homme du métier est à même de trouver le compromis optimal entre, d'une part, une efficacité maximale de ce composé, qui a un effet bénéfique sur l'activité et la stabilité du catalyseur, et d'autre part, des considérations économiques liées au coût entraîné par le recyclage dans le procédé de ce composé.

Le second élément du système catalytique mis en oeuvre dans le procédé selon l'invention est constitué par au moins un composé à base d'iridium.

Tout d'abord, la réaction selon l'invention est plus particulièrement mise en oeuvre en présence d'un catalyseur homogène. En d'autres termes, cela signifie que le composé à base d'iridium notamment se trouve sous une forme soluble dans le mélange réactionnel. Il est à noter que la présence d'une partie dudit composé à base d'iridium sous une forme non solubilisée, ne présente pas de difficulté majeure pour la mise en oeuvre de la réaction.

Tous les composés de l'iridium solubles ou pouvant être solubilisés dans le milieu réactionnel, dans les conditions de réalisation de l'invention, peuvent être utilisés. A titre d'exemple et sans intention de se limiter, conviennent notamment à la mise en oeuvre de l'invention, l'iridium à l'état métallique, les sels simples de ce métal, les oxydes ou encore les complexes de coordination.

En tant que sels simples d'iridium, on utilise habituellement les halogénures d'iridium. L'halogène est plus particulièrement choisi parmi le chlore, le brome ou l'iode, ce dernier étant préféré. Ainsi les composés comme Irl₃, IrBr₃, IrCl₃, Irl₃.4H₂O, Irl₄, IrBr₃.4H₂O peuvent être utilisés dans le procédé selon l'invention.

Les oxydes choisis parmi IrO₂, Ir₂O₃. xH₂O peuvent de même être convenablement mis en oeuvre dans le procédé selon l'invention.

En ce qui concerne les complexes solubles de coordination de l'iridium, les composés les plus couramment mis en oeuvre sont ceux présentant des ligands choisis parmi le monoxyde de carbone ou une combinaison monoxyde de carbone/halogène, l'halogène étant choisi parmi le chlore, le brome ou plus particulièrement l'iode. Il n'est toutefois pas exclu d'utiliser des complexes solubles d'iridium dont les ligands sont choisis parmi des composés organo-phosphorés ou organo-azotés, par exemple.

En tant que complexes de coordination, connus de l'homme du métier, convenant particulièrement à la mise en oeuvre de l'invention, on peut citer sans intention de se limiter les composés suivants : Ir₄(CO)₁₂, Ir(CO)₂I₂-Q⁺, Ir(CO)₂Br₂⁻Q⁺, Ir(CO)₂Cl₂⁻Q⁺ ; formules dans lesquelles Q peut représenter notamment l'hydrogène, le groupe NR₄, PR₄, avec R choisi parmi l'hydrogène, un radical hydrocarboné.

Ces catalyseurs peuvent être obtenus par toute méthode connue de l'homme du métier. Ainsi, on pourra se reporter aux demandes de brevets EP 657 386 et WO 95/17963 pour la préparation de solutions catalytiques à base d'iridium convenant à la réalisation de la présente invention.

Il est à noter que la réaction selon l'invention peut être mise en oeuvre avec un système catalytique comprenant, outre l'iridium, un ou plusieurs autres métaux du groupe VIII. Plus particulièrement, la réaction peut être mise en oeuvre avec une association de rhodium et d'iridium, ou encore une association de ruthénium et d'iridium, de rhénium et d'iridium, ou encore un système catalytique à base d'une combinaison quelconque de ces quatre métaux.

Si une telle variante est adoptée, le rapport molaire de l'iridium aux autres métaux associés est plus particulièrement compris entre 1/10 à 10/1. De préférence, il est supérieur à 1/1.

Généralement, la concentration totale en iridium dans le milieu réactionnel est comprise entre 0,1 et 100 mmol/l de préférence entre 1 et 25 mmol/l.

Ainsi que cela a été indiqué auparavant, la réaction est mise en oeuvre en alimentant des réactifs apportant des radicaux formyles et des radicaux méthyles.

Par réactifs apportant des radicaux formyles, on désigne des réactifs de formule HC(O)OR, formule dans laquelle R représente un atome d'hydrogène ou un méthyle. Selon un mode de réalisation particulier de l'invention, on alimente du formiate de méthyle, sachant qu'il n'est bien entendu pas exclu d'alimenter de l'acide formique ou un mélange d'acide et d'ester.

Par réactifs apportant des radicaux méthyles, on définit des réactifs de formule CH₃-R', dans laquelle -R' représente -OH, -OCH₃,-OC(O)CH₃, -OC(O)H. Là encore, on ne sortirait pas du cadre de l'invention en alimentant plusieurs des réactifs apportant les radicaux méthyles précités.

Il est à noter que dans le cas du formiate de méthyle, la molécule introduite comprend à la fois un radical méthyle et un radical formyle. Dans ce cas, la molécule comprend chacun des deux radicaux.

Il est aussi à noter que, dans le cas où on alimente la réaction en diméthyléther CH₃-O-CH_{3,} la molécule introduite comporte deux radicaux méthyles.

Par radicaux méthyles et formyles alimentés dans la réaction on entend les radicaux méthyles et formyles consommés par cette réaction. Ainsi, ne sont pas compris les radicaux méthyles et formyles recyclés, si le procédé est mis en oeuvre en continu, ou bien encore les radicaux restant dans le milieu réactionnel à la fin de la réaction, si celle-ci est conduite en discontinu.

Comme cela a été mentionné auparavant, la réaction est mise en oeuvre en alimentant les radicaux méthyles et formyles dans un rapport molaire supérieur à 1.

La valeur de ce rapport permet de fixer les réactions qui sont mises en jeu.

Le fait que ce rapport soit supérieur à 1 rend les conditions favorables pour l'existence des deux réactions d'isomérisation et de carbonylation.

Le rapport molaire des radicaux méthyles aux radicaux formyles peut varier dans de larges limites. Il peut ainsi être compris entre 1 et 100. Selon un mode réalisation particulier de l'invention, le rapport molaire précité est compris entre 1 exclu et 20 inclus.

La réaction d'isomérisation et de carbonylation selon l'invention est mise en oeuvre en présence d'eau et d'un solvant.

La quantité d'eau, exprimée en poids du mélange réactionnel, varie entre 0 exclu et 5 %. D'une manière avantageuse, ladite teneur est comprise entre 0 exclu et 2 % en poids.

Il est à noter que l'eau joue un rôle important dans le procédé. En effet, celle-ci participe au maintien en solution du catalyseur, en particulier dans la zone de vaporisation partielle du mélange (flash) qui sera décrite ultérieurement. Elle permet aussi de limiter les réactions secondaires connues pour les procédés mis en oeuvre dans des conditions anhydres.

En ce qui concerne le solvant, celui-ci peut comprendre un ou plusieurs acides carboxyliques ainsi que d'autres composés dénommés co-solvants dans le présent mémoire.

Selon un mode de réalisation particulier de la présente invention, l'acide carboxylique est choisi parmi les acides aliphatiques présentant de 2 à 10 atomes de carbone, de préférence comprenant de 2 à 5 atomes de carbone. Selon un mode de réalisation particulièrement avantageux de la présente invention, ledit acide carboxylique est l'acide acétique. On ne sortirait pas du cadre de la présente invention en employant un mélange d'acides précités.

Selon une autre possibilité, le solvant peut comprendre en outre de l'acide formique qui est compté, par ailleurs, comme acide carboxylique.

Bien entendu, on ne sortirait pas du cadre de la présente invention en mettant en oeuvre un solvant supplémentaire (co-solvant), inerte dans les conditions de réaction. A titre d'exemple de ce type de solvant, on peut citer les esters, les éthers, les cétones, les amides, les sulfoxydes ou encore les hydrocarbures. Le co-solvant préféré est l'ester de l'acide produit, soit l'acétate de méthyle.

Ainsi, une variante particulièrement avantageuse de l'invention consiste à utiliser, en tant que solvant, un mélange comprenant l'acide produit, éventuellement l'ester correspondant à l'acide produit, et l'acide formique.

La quantité de solvant mise en jeu dans la réaction correspond au complément à 100 % en poids du mélange réactionnel.

Si un ou plusieurs co-solvants sont employés, la quantité d'acide carboxylique est de préférence supérieure ou égale à celle du co-solvant. Ainsi, dans le cas où le co-solvant est l'acétate de méthyle, la teneur pondérale de celui-ci est de préférence inférieure ou égale à celle de l'acide acétique.

Selon un mode de réalisation particulier de l'invention, la quantité d'acide formique présent dans le milieu réactionnel est maintenue inférieure à 15 % en poids du mélange réactionnel. De préférence, la teneur en acide formique est maintenue inférieure à 12 % et plus particulièrement inférieure à 10 % en poids du mélange réactionnel.

Par ailleurs, selon un mode de réalisation avantageux de la présente invention, la quantité d'acides carboxyliques libres présents dans le mélange réactionnel est supérieure à 25 % en poids dudit mélange et telle que la totalité des constituants du mélange réactionnel représente 100 % en poids du mélange réactionnel. Plus particulièrement, la quantité en acides carboxyliques libres est supérieure à 30 % en poids du mélange réactionnel, et de préférence, elle est supérieure à 40 % en poids du mélange réactionnel.

Il est à noter que le rapport molaire acide formique /acétate de méthyle peut être différent de 1 dans les conditions de la réaction, c'est-à-dire supérieur ou inférieur à cette valeur. On peut bien évidemment effectuer la réaction avec un rapport molaire égal à 1.

Le procédé de l'invention consiste donc à maintenir pendant la durée de la réaction, une pression partielle en monoxyde de carbone et une concentration en réactif apportant les radicaux formyles bien déterminées.

Ainsi, la pression partielle en monoxyde de carbone est maintenue comprise entre 0,1.10⁵ Pa et 25.10⁵ Pa.

Les pressions sont exprimées en pascals absolus, et ont été mesurées à chaud, c'est-à-dire dans les conditions de température de la réaction.

Selon un mode de réalisation plus particulier de l'invention, on maintient une pression partielle en monoxyde de carbone supérieure à 0,5.10⁵ Pa, et de préférence supérieure à 10⁵ Pa.

La pression partielle en monoxyde de carbone, de manière avantageuse, est inférieure à 15.10⁵ Pa. Plus particulièrement, elle est inférieure à 10.10⁵ Pa.

Lorsque les conditions sont favorables à la fois pour la réaction d'isomérisation et celle de carbonylation, on constate qu'il y a une consommation de ce gaz, due à la réaction de carbonylation, consommation qu'il est nécessaire de compenser pour conserver la pression partielle en monoxyde de carbone dans les gammes requises précitées.

Il est à noter que le monoxyde de carbone peut être utilisé sous forme pure ou diluée dans des gaz tels que l'hydrogène, le méthane, le dioxyde de carbone ou tout autre type de gaz comme par exemple l'azote. De préférence, on utilise un monoxyde de carbone pur à au moins 98% en volume.

Selon une caractéristique de la présente invention, la teneur en réactif apportant des radicaux formyles est maintenue inférieure à 20 % en poids du mélange réactionnel.

De manière préférée, la teneur en réactif précité ne dépasse pas 10 % en poids du mélange réactionnel. Selon un mode de réalisation particulièrement avantageux de la présente invention, la teneur en réactif précité ne dépasse pas 5 % en poids du mélange réactionnel.

Lorsque la réaction est mise en oeuvre en continu, les caractéristiques précitées sont de préférence maintenues constantes pendant le cours de la réaction, mais une évolution de la pression partielle en monoxyde de carbone est possible pendant la réaction, dans la mesure où elle se trouve toujours comprise dans la gamme précitée.

Lorsque la réaction est mise en oeuvre en discontinu, la quantité en réactif apportant des radicaux formyles est maintenue inférieure aux valeurs indiquées bien qu'évoluant en décroissant, car ledit réactif est consommé par la réaction. Quant à la pression partielle en monoxyde de carbone, elle peut ou non être maintenue constante, à la condition de se trouver dans la plage de valeurs indiquées.

La réaction est en général mise en oeuvre à une température comprise entre 150 et 250°C. Plus particulièrement la température de réaction est comprise entre 175 et 210°C. De préférence, elle est comprise entre 175 et 200°C.

La pression totale sous laquelle est conduite la réaction est en général supérieure à la pression atmosphérique. Plus particulièrement elle est inférieure à 100.10⁵ Pa et de préférence inférieure ou égale à 50.10⁵ Pa. Les pressions sont exprimées en pascals absolus, et ont été mesurées à chaud, c'est-à-dire dans les conditions de température de la réaction.

La réaction d'isomérisation et de carbonylation faisant l'objet de l'invention est de préférence mise en oeuvre en présence d'une teneur en métaux de corrosion inférieure à 2000 ppm. Les métaux de corrosion sont notamment le fer, le nickel, le chrome, le molybdène, et éventuellement le zirconium. La teneur en métaux de corrosion dans le mélange réactionnel est maintenue par les méthodes connues de l'homme du métier, comme par exemple la précipitation sélective, l'extraction liquide, le passage sur des résines échangeuses d'ions.

La réaction est mise en oeuvre dans des appareillages résistant à la corrosion créée par le milieu. Ainsi, le zirconium ou encore les alliages du type Hastelloy® C ou B, conviennent particulièrement bien aux conditions de mise en oeuvre de la réaction.

Lors du démarrage de la réaction, les divers composants sont introduits dans un réacteur approprié, muni de moyens d'agitation pour assurer une bonne homogénéité du mélange réactionnel. Il est à noter que si le réacteur comprend de préférence des moyens d'agitation mécanique du mélange réactionnel, il n'est pas exclu d'opérer sans de tels moyens, l'homogénéisation du mélange pouvant être réalisée par l'introduction du monoxyde de carbone dans le réacteur.

Il est à noter que la réaction pourrait être convenablement mise en oeuvre dans un réacteur de type piston.

La combinaison de plusieurs réacteurs de type agité et piston est bien entendu envisageable.

L'introduction de monoxyde de carbone peut avoir lieu directement dans le réacteur où a lieu la réaction selon l'invention, mais aussi dans la zone de recyclage qui sera décrite plus bas.

Le mélange réactionnel en sortie du réacteur où a lieu la réaction selon l'invention, est traité de manière appropriée pour séparer les produits du mélange réactionnel comprenant notamment le catalyseur.

A ce titre, et dans le cas de mise en oeuvre de la réaction en continu, on peut employer par exemple, une technique classique consistant à détendre le mélange de manière à provoquer une vaporisation partielle de ce dernier. Cette opération est mise en oeuvre grâce à une vanne permettant de détendre le mélange, ce dernier étant par la suite introduit dans un séparateur (dit séparateur flash). L'opération de détente du mélange peut avoir lieu avec ou, de préférence, sans apport de chaleur, c'est-à-dire dans des conditions adiabatiques.

Selon une variante de l'invention, la teneur en eau dans la zone de vaporisation partielle est maintenue à une valeur d'au moins 0,5 % en poids par rapport au poids de la partie non vaporisée. Ceci peut avoir lieu, si nécessaire, par injection d'eau dans ladite zone de vaporisation partielle, c'est-à-dire dans le séparateur flash.

La partie vaporisée comprenant, entre autres, l'acide acétique et/ou l'acétate de méthyle produits peut être mise en contact et lavée dans la partie supérieure du séparateur flash par un liquide provenant des installations de purification en aval.

En sortie du séparateur flash, la partie non vaporisée comprenant notamment le catalyseur resté en solution, pour tout ou partie, est recyclée de manière avantageuse au réacteur, classiquement au moyen d'une pompe.

La partie vaporisée, comprenant en outre l'acide acétique et/ou l'acétate de méthyle produits, est ensuite envoyée dans une zone de purification, comprenant, de manière habituelle, diverses colonnes de distillation.

Selon une variante de l'invention, une mise en contact et un lavage complémentaire peuvent être réalisés dans la première colonne de distillation à l'aide d'un liquide provenant des installations de purification.

Selon une autre variante de l'invention, le mélange réactionnel en sortie du réacteur peut être directement détendu dans la première colonne de distillation de la zone de purification.

Les différents flux de matière séparés dans la zone de purification peuvent être recyclés vers le réacteur ou traités de façon indépendante.

L'acide acétique ou l'acétate de méthyle obtenu par ce procédé est de qualité suffisante pour être vendu sans purification autre que celles connues de l'homme de l'art et déjà dans le domaine public.

L'introduction de monoxyde de carbone peut avoir lieu directement dans le réacteur, mais aussi dans la zone de recyclage de la fraction liquide non vaporisée, de telle sorte que le monoxyde de carbone ne soit pas dégazé directement vers la zone de vaporisation partielle du mélange réactionnel. Dans ce but, l'introduction du monoxyde de carbone selon cette dernière possibilité, est plus particulièrement effectuée en aval de la pompe de recyclage du mélange réactionnel.

Selon une autre variante de l'invention, et dans le but de minimiser les pertes en monoxyde de carbone, le mélange réactionnel en sortie du réacteur dans lequel a lieu la réaction selon l'invention est introduit, avant l'étape de vaporisation partielle, dans un réacteur supplémentaire. Cette étape supplémentaire a pour résultat de consommer le monoxyde de carbone dissous dans ledit mélange réactionnel, pour donner l'acide souhaité. De ce fait, les pertes en monoxyde de carbone lors de la vaporisation partielle du mélange réactionnel sont considérablement limitées.

Le procédé de l'invention, tel qu'il a été décrit précédemment et dans toutes ses variantes, s'applique tout particulièrement à la préparation d'acide acétique et/ou d'acétate de méthyle à partir de mélanges de formiate de méthyle et de méthanol et, ceci, indépendamment des proportions relatives de ces deux réactifs.

Ainsi, l'invention concerne également un procédé de préparation d'acide acétique et/ou d'acétate de méthyle par réaction de méthanol et de formiate de méthyle, en présence d'eau, de monoxyde de carbone, d'un solvant et d'un système catalytique comprenant au moins un promoteur halogéné et au moins un composé à base d'iridium, caractérisé en ce qu'il s'agit d'un procédé continu dans lequel on maintient une pression partielle en monoxyde de carbone comprise entre 0,1.10⁵ Pa et 25.10⁵ Pa, une quantité de formiate de méthyle inférieure à 20 % en poids du mélange réactionnel et une quantité d'eau comprise entre 0 exclu et 5 % en poids du mélange réactionnel.

Les proportions de formiate de méthyle et de méthanol pourront varier dans un très large éventail. D'une façon générale, on choisira un rapport pondéral de réactif méthanol au réactif formiate de méthyle compris entre 0,01 et 100 inclus.

Selon ce procédé, le système réactionnel est caractérisé de la façon suivante :
- les réactifs sont le formiate de méthyle, le méthanol et le monoxyde de carbone ;
- le système catalytique comprend l'iridium sous forme soluble et au moins un promoteur, avantageusement l'iodure de méthyle ;
- l'eau ;
- le solvant comprend l'acide acétique, l'acide formique et avantageusement un co-solvant, l'acétate de méthyle.

Selon un mode préféré de réalisation du procédé ci-dessus, on alimente en continu du formiate de méthyle et du méthanol dans un réacteur muni de moyens d'agitation.

Du monoxyde de carbone d'une pureté d'au moins 98 % est injecté dans le réacteur en quantité adéquate.

La température est maintenue avantageusement à une valeur comprise entre 175°C et 200°C.

La pression est maintenue avantageusement à une valeur comprise entre 20 et 30 bars absolus.

Un flux gazeux est soutiré en continu en tête du réacteur pour maintenir la pression partielle de monoxyde de carbone à une valeur comprise entre 1 et 10 bars. Il est traité et débarrassé des sous-produits gazeux de la réaction dans une section classique de traitement des évents gazeux.

Le mélange réactionnel sortant du réacteur est détendu dans un séparateur flash. La partie non vaporisée est recyclée au réacteur au moyen d'une pompe. La partie vaporisée est envoyée dans une zone de purification comprenant diverses colonnes de distillation.

Des flux liquides provenant de diverses zones de purification et du traitement des évents gazeux et comprenant de l'acide acétique, de l'eau, du méthanol, du formiate de méthyle, de l'acétate de méthyle, du catalyseur iridium et son promoteur l'iodure de méthyle et de l'acide formique sont classiquement recyclés vers le réacteur.

Le procédé est très sélectif et ne génère que peu de sous-produits. On peut citer à titre illustratif les principaux sous-produits :
- gazeux : méthane, dioxyde de carbone et hydrogène ;
- et liquides : acide propionique.

D'autres sous-produits peuvent être générés à des teneurs faibles et sont éliminés selon les procédés connus de l'homme de l'art dans la zone de purification.

L'acide acétique produit est de qualité suffisante pour être vendu.

Le rapport des matières premières alimentant le réacteur peut varier dans de larges proportions. Ainsi, en alimentant le réacteur avec du formiate de méthyle de qualité commerciale contenant quelques pour-cent de méthanol, la consommation de monoxyde de carbone est faible.

L'acide acétique est produit principalement par l'isomérisation du formiate de méthyle et de façon plus réduite par la carbonylation du méthanol.

Un autre cas de figure est d'assurer un complément de production à un système réactionnel consommant principalement du méthanol et du monoxyde de carbone. L'apport de formiate de méthyle permet d'assurer une diversification des matières premières et génère une quantité supplémentaire d'acide acétique produit.

Le rapport en poids de méthanol alimentant le réacteur au formiate de méthyle alimentant le réacteur peut varier de 0,01 à 100.

Ainsi, ce rapport est défini en terme de réactif méthanol par rapport au réactif formiate de méthyle au titre de la présente invention.

Des exemples concrets mais non limitatifs de l'invention vont maintenant être présentés.

### EXEMPLE 1

### Réaction d'isomérisation et de carbonylation sans recyclage de catalyseur

Tout d'abord, la solution catalytique est préparée comme suit :

On introduit dans un autoclave :
* 105 g d'iodure d'iridium,
* 90 g d'acide iodhydrique en solution à 57 % dans l'eau
* 910 g d'acide acétique.

L'autoclave est ensuite pressurisé avec 50.10⁵ Pa (50 bars) de monoxyde de carbone.

On porte la température à 150°C.

La durée de la réaction est de 4 heures.

L'autoclave est ensuite dépressurisé puis le milieu réactionnel refroidi.

On obtient une solution de couleur rouge, que l'on décante pour obtenir la solution catalytique.

Dans un autoclave en Hastelloy® B2, on introduit en continu une solution d'iridium dans l'acide acétique telle que préparée ci-dessus, de l'acide acétique, de l'iodure de méthyle, du formiate de méthyle, du méthanol et de l'eau.

Le rapport molaire des radicaux méthyles alimentés aux radicaux formyles alimentés est de 2,4.

La composition du mélange réactionnel en régime stabilisé est la suivante:

| | |
|---|---|
| eau | 1,13% |
| acétate de méthyle | 25,8% |
| iodure de méthyle | 7,3 % |
| acide formique | 13,0 % |
| formiate de méthyle | 17,7 % |
| méthanol | 0,29 % |
| acide acétique | complément à 100 % |
| la concentration en iridium est de | 3080 ppm |

La pression partielle en monoxyde de carbone est maintenue constante à une valeur de 10⁵ Pa (1 bar).

La pression totale en sortie du réacteur est de 25.10⁵ Pa (25 bars).

La température est maintenue à 190°C ± 0,5°C.

La composition du mélange réactionnel, en pour-cent massique, donnée avec une précision de l'ordre de 2%, est déterminée par dosage en chromatographie en phase vapeur.

Le calcul de la vitesse d'isomérisation est effectué sur le liquide issu du réacteur, refroidi à température ambiante et, collecté pendant une durée comprise entre 30 et 60 minutes par rapport aux flux des composés injectés dans le réacteur pendant cette même durée, après que le régime chimique stabilisé a été obtenu.

Le calcul de la vitesse de carbonylation est fait d'après le bilan gaz sur le monoxycle de carbone consommé..

On obtient une vitesse d'isomérisation de 8 mol/h/l en acide acétique formé, et une vitesse de carbonylation de 11 mol/h/l en acide acétique formé. L'acide acétique se trouve sous la forme d'acide acétique et d'acétate de méthyle.

### EXEMPLE 2

### Réaction d'isomérisation avec carbonylation avec recyclage de catalyseur

Dans un autoclave en Hastelloy® B2 contenant du catalyseur préparé selon la méthode décrite dans l'exemple 1, on injecte en continu les différents composants du mélange réactionnel : acide acétique, formiate de méthyle, rnéthanol, acétate de méthyle, iodure de méthyle, et éventuellement eau. Les flux sortant du réacteur sont dirigés dans une zone où une fraction contenant l'acide acétique produit est vaporisée. La fraction non vaporisée contenant le catalyseur est recyclée au réacteur. La fraction vaporisée est condensée et représente les effluents liquides.

La composition du mélange réactionnel en régime stabilisé, déterminée par dosage en chromatographie en phase vapeur d'un échantillon prélevé du milieu réactionnel, exprimée en pourcentages massiques, est la suivante :

| | |
|---|---|
| eau | 1,25 % |
| méthanol | 0,15 % |
| acétate de méthyle | 18,3 % |
| iodure de méthyle | 9,7 % |
| acide formique | 5,9 % |
| formiate de méthyle | 3,0 % |
| acide acétique | complément à 100 % |
| la concentration en iridium est de | 2180 ppm |

La température est maintenue à 190°C +/- 0,5°C.

La pression totale du réacteur est maintenue à 2,4 MPa +/- 20 kPa (24 bars).

La pression partielle de monoxyde de carbone est maintenue constante à une valeur de 1,05 MPa (10,5 bars) ; le CO utilisé est de pureté supérieure à 99 %.

Le calcul de la vitesse de formation de l'acide acétique par les deux réactions d'isomérisation du formiate de méthyle et de carbonylation du méthanol est effectué grâce au bilan sur les effluents liquides de la zone de vaporisation, collectés pendant une durée donnée (comprise entre 2 et 4 h), par rapport aux flux des composés injectés pendant le même laps de temps, après que le régime chimique a été stabilisé. Le calcul de la vitesse de carbonylation est fait d'après le bilan gaz sur le monoxyde de carbone consommé.

On obtient une vitesse d'isomérisation de 1,2 mol.h⁻¹I⁻¹ en acide acétique formé, et une vitesse de carbonylation de 15,7 mol.h⁻¹I⁻¹ en acide acétique formé. L'acide acétique se trouve sous la forme d'acide acétique et d'acétate de méthyle.

Le rapport molaire des radicaux méthyles alimentés aux radicaux formyles alimentés est de 14,1.

## Revendications

1. Procédé de préparation d'acide acétique et/ou d'acétate de méthyle par réaction de réactifs apportant des radicaux formyles et de réactifs apportant des radicaux méthyles, en présence de monoxyde de carbone, d'eau, d'un solvant et d'un système catalytique comprenant au moins un promoteur halogéné et au moins un composé à base d'iridium, **caractérisé en ce que** ledit procédé est un procédé continu dans lequel on maintient une pression partielle en monoxyde de carbone comprise entre 0,1.10⁵ Pa et 25.10⁵ Pa, une quantité en réactif apportant les radicaux formyles inférieure ou égale à 20 % en poids du mélange réactionnel, on alimente les réactifs apportant des radicaux méthyles et formyles dans un rapport molaire des radicaux méthyles aux radicaux formyles supérieur à 1 et on maintient une quantité d'eau comprise entre 0 exclu et 5 % en poids du mélange réactionnel.

2. Procédé selon la revendication 1, **caractérisé en ce que** lesdits réactifs apportant des radicaux formyles sont des produits de formule HC(O)OR, dans laquelle R représente un atome d'hydrogène ou un méthyle.

3. Procédé selon la revendication 1 ou 2, **caractérisé en ce que** lesdits réactifs apportant des radicaux méthyles répondent à la formule CH₃-R', formule dans laquelle -R' représente -OH, -OCH₃, -OC(O)CH₃, -OC(O)H, ces réactifs étant utilisés seuls ou en mélange.

4. Procédé selon l'une quelconque des revendications 1 à 3, **caractérisé en ce qu'**il est mis en oeuvre avec un rapport molaire des radicaux méthyles aux radicaux formyles compris entre 1 exclu et 100, de préférence entre 1 exclu et 20 inclus.

5. Procédé de préparation d'acide acétique et/ou d'acétate de méthyle par réaction de méthanol et de formiate de méthyle, en présence d'eau, de monoxyde de carbone, d'un solvant et d'un système catalytique comprenant au moins un promoteur halogéné et au moins un composé à base d'iridium, **caractérisé en ce qu'**il s'agit d'un procédé continu dans lequel on maintient une pression partielle en monoxyde de carbone comprise entre 0,1.10⁵ Pa et 25.10⁵ Pa, une quantité de formiate de méthyle inférieure à 20 % en poids du mélange réactionnel et une quantité d'eau comprise entre 0 exclu et 5 % en poids du mélange réactionnel.

6. Procédé selon la revendication 5, **caractérisé en ce qu'**il est mis en oeuvre avec un rapport pondéral de réactif méthanol alimentant le réacteur au réactif formiate de méthyle alimentant le réacteur compris entre 0,01 et 100 inclus.

7. Procédé selon l'une quelconque des revendications 1 à 6, **caractérisé en ce qu'**il est mis en oeuvre en maintenant une pression partielle en monoxyde de carbone supérieure ou égale à 0,5.10⁵ Pa, de préférence supérieure ou égale à 10⁵ Pa.

8. Procédé selon l'une quelconque des revendications 1 à 7, **caractérisé en ce qu'**il est mis en oeuvre en maintenant une pression partielle en monoxyde de carbone inférieure ou égale à 15.10⁵ Pa, de préférence inférieure ou égale à 10.10⁵ Pa.

9. Procédé selon l'une quelconque des revendications 1 à 8, **caractérisé en ce qu'**il est mis en oeuvre en maintenant une quantité d'eau comprise entre 0 exclu et 2 % en poids du mélange réactionnel.

10. Procédé selon l'une quelconque des revendications 1 à 9, **caractérisé en ce qu'**il est mis en oeuvre en maintenant une quantité de promoteur halogéné comprise entre 0,1 et 20 % en poids du mélange réactionnel, de préférence comprise entre 1 et 15 % en poids du mélange réactionnel.

11. Procédé selon l'une quelconque des revendications 1 à 10, **caractérisé en ce qu'**il est mis en oeuvre en présence d'un solvant qui est choisi parmi les acides carboxyliques aliphatiques comportant 2 à 10 atomes de carbone.

12. Procédé selon l'une quelconque des revendications 1 à 11, **caractérisé en ce qu'**il est mis en oeuvre en présence d'acide formique à une teneur maintenue inférieure à 15 % en poids du mélange réactionnel, et de préférence inférieure à 12 % en poids du mélange réactionnel.

13. Procédé selon la revendication 12, **caractérisé en ce que** la teneur en acide formique est maintenue inférieure à 10 % en poids du mélange réactionnel.

14. Procédé selon l'une quelconque des revendications 11 à 13, **caractérisé en ce que** la quantité d'acides carboxyliques libres présents dans le mélange réactionnel est supérieure à 25 % en poids dudit mélange.

15. Procédé selon l'une quelconque des revendications 1 à 14, **caractérisé en ce qu'**il est mis en oeuvre en présence d'un co-solvant qui est l'acétate de méthyle.

16. Procédé selon la revendication 15, **caractérisé en ce que** la teneur pondérale en co-solvant est inférieure ou égale à celle de l'acide acétique.

17. Procédé selon l'une quelconque des revendications 1 à 16, **caractérisé en ce qu'**il est mis en oeuvre en présence d'un promoteur halogéné choisi parmi les composés iodés ou un précurseur de tels composés.

## Patentansprüche

1. Verfahren zur Herstellung von Essigsäure und/oder Methylacetat durch Umsetzung von Reagentien, die Formylreste aufweisen, und von Reagentien, die Methylreste aufweisen, in Gegenwart von Kohlenmonoxid, Wasser, eines Lösungsmittels und eines katalytischen Systems, das mindestens einen halogenierten Promotor und mindestens eine Verbindung auf Iridiumbasis umfasst, **dadurch gekennzeichnet, dass** das genannte Verfahren ein kontinuierliches Verfahren ist, in dem man einen Kohlenmonoxid-Partialdruck zwischen 0,1.10⁵ und 25.10⁵ Pa und eine Menge des Reagens, welches die Formylreste aufweist, von ≤ 20 Gew.-%, bezogen auf das Gewicht der Reaktionsmischung, aufrechterhält, die Reagentien, die Methylreste und Formylreste aufweisen, in einem Molverhältnis zwischen den Methylresten und den Formylresten von größer als 1 zuführt und eine Wassermenge aufrechterhält, die zwischen > 0 und 5 Gew.-%, bezogen auf das Gewicht der Reaktionsmischung, liegt.

2. Verfahren nach Anspruch 1, **dadurch gekennzeichnet, dass** die genannten Reagentien, die Formylreste aufweisen, Produkte der Formel HC(O)OR sind, worin R für ein Wasserstoffatom oder eine Methylgruppe steht.

3. Verfahren nach Anspruch 1 oder 2, **dadurch gekennzeichnet, dass** die genannten Reagentien, die Methylreste aufweisen, der Formel CH₃-R' entsprechen, in der -R' steht für -OH, -OCH₃, -OC(O)CH₃ und -OC(O)H, wobei diese Reagentien einzeln oder in Form einer Mischung verwendet werden.

4. Verfahren nach einem der Ansprüche 1 bis 3, **dadurch gekennzeichnet, dass** man es mit einem Molverhältnis zwischen den Methylresten und den Formylresten zwischen > 1 und 100, vorzugsweise zwischen > 1 und ≤ 20, durchführt.

5. Verfahren zur Herstellung von Essigsäure und/oder Methylacetat durch Umsetzung von Methanol und Methylformiat in Gegenwart von Wasser, Kohlenmonoxid, eines Lösungsmittels und eines katalytischen Systems, das mindestens einen halogenierten Promotor und mindestens eine Verbindung auf Iridiumbasis umfasst, **dadurch gekennzeichnet, dass** es sich handelt um ein kontinuierliches Verfahren, in dem man einen Kohlenmonoxid-Partialdruck zwischen 0,1.10⁵ und 25.10⁵ Pa, eine Methylformiat-Menge von < 20 Gew.-%, bezogen auf das Gewicht der Reaktionsmischung, und eine Wassermenge zwischen > 0 und 5 Gew.-%, bezogen auf das Gewicht der Reaktionsmischung, aufrechterhält.

6. Verfahren nach Anspruch 5, **dadurch gekennzeichnet, dass** es mit einem Gewichtsverhältnis zwischen dem dem Reaktor zugeführten Reagens Methanol und dem dem Reaktor zugeführten Reagens Methylformiat zwischen 0,01 und ≤ 100 durchgeführt wird.

7. Verfahren nach einem der Ansprüche 1 bis 6, **dadurch gekennzeichnet, dass** man es durchführt unter Aufrechterhaltung eines Kohlenmonoxid-Partialdruckes von ≥ 0,5.10⁵ Pa, vorzugsweise ≥ 10⁵ Pa.

8. Verfahren nach einem der Ansprüche 1 bis 7, **dadurch gekennzeichnet, dass** man es durchführt unter Aufrechterhaltung eines Kohlenmonoxid-Partialdruckes von ≤ 15.10⁵ Pa, vorzugsweise von ≤ 10.10⁵ Pa.

9. Verfahren nach einem der Ansprüche 1 bis 8, **dadurch gekennzeichnet, dass** man es durchführt, indem man eine Wassermenge zwischen > 0 und 2 Gew.-%, bezogen auf das Gewicht der Reaktionsmischung, aufrechterhält.

10. Verfahren nach einem der Ansprüche 1 bis 9, **dadurch gekennzeichnet, dass** man es durchführt, indem man eine Menge an halogeniertem Promotor zwischen 0,1 und 20 Gew.-%, bezogen auf das Gewicht der Reaktionsmischung, vorzugsweise zwischen 1 und 15 Gew.-%, bezogen auf das Gewicht der Reaktionsmischung, aufrechterhält.

11. Verfahren nach einem der Ansprüche 1 bis 10, **dadurch gekennzeichnet, dass** man es durchführt in Gegenwart eines Lösungsmittels, das ausgewählt wird unter den aliphatischen Carbonsäuren mit 2 bis 10 Kohlenstoffatomen.

12. Verfahren nach einem der Ansprüche 1 bis 11, **dadurch gekennzeichnet, dass** man es durchführt in Gegenwart von Ameisensäure, deren Gehalt bei < 15 Gew.-%, bezogen auf das Gewicht der Reaktionsmischung, vorzugsweise < 12 Gew.-%, bezogen auf das Gewicht der Reaktionsmischung, gehalten wird.

13. Verfahren nach Anspruch 12, **dadurch gekennzeichnet, dass** der Ameisensäure-Gehalt bei < 10 Gew.-%, bezogen auf das Gewicht der Reaktionsmischung, gehalten wird.

14. Verfahren nach einem der Ansprüche 11 bis 13, **dadurch gekennzeichnet, dass** die Menge der in der Reaktionsmischung vorliegenden freien Carbonsäuren > 25 Gew.-%, bezogen auf das Gewicht der Mischung, beträgt.

15. Verfahren nach einem der Ansprüche 1 bis 14, **dadurch gekennzeichnet, dass** man es in Gegenwart eines Colösungsmittels durchführt, bei dem es sich um Methylacetat handelt.

16. Verfahren nach Anspruch 15, **dadurch gekennzeichnet, dass** der Gewichtsgehalt an Colösungsmittel gleich demjenigen an Essigsäure oder niedriger ist.

17. Verfahren nach einem der Ansprüche 1 bis 16, **dadurch gekennzeichnet, dass** es in Gegenwart eines halogenierten Promotors durchgeführt wird, der ausgewählt wird unter den iodierten Verbindungen oder einem Verläufer dieser Verbindungen.

## Claims

1. Method of preparing acetic acid and/or methyl acetate by reaction of reagents which provide formyl radicals and reagents which provide methyl radicals, in the presence of carbon monoxide, water, a solvent and a catalytic system comprising at least one halogenated promoter and at least one iridium-based compound, **characterised in that** said method is a method carried out continuously wherein a partial carbon monoxide pressure is kept between 0.1 . 10⁵ Pa and 25 . 10⁵ Pa, an amount of reagent which provides the formyl radicals is kept below or equal to 20% by weight of the reaction mixture, the reagents which provide the methyl radicals and formyl radicals are fed in in a molar ratio of the methyl radicals to the formyl radicals of greater than 1, and an amount of water between 0 (excluded) and 5 % by weight of the reaction mixture is maintained.

2. Method according to claim 1, **characterised in that** said reagents which provide formyl radicals are products of the formula HC(O)OR, in which R represents a hydrogen atom or a methyl group.

3. Method according to claim 1 or 2, **characterised in that** said reagents which provide methyl radicals are of the formula CH₃·R', in which formula -R' represents -OH, ·OCH₃, -OC(O)CH₃, -OC(O)H, these reagents being used alone or in a mixture.

4. Method according to any one of claims 1 to 3, **characterised in that** the reaction is carried out with a molar ratio of the methyl radicals to the formyl radicals of between 1 (excluded) and 100, preferably between 1 (excluded) and 20 (included).

5. Method of preparing acetic acid and/or methyl acetate by reaction of methanol and methyl formate, in the presence of water, carbon monoxide, a solvent and a catalytic system comprising at least one halogenated promoter and at least one iridium-based compound, **characterised in that** said method is a continuous method wherein a partial carbon monoxide pressure is kept between 0.1 . 10⁵ Pa and 25 . 10⁵ Pa, an amount of methyl formate is kept below 20% by weight of the reaction mixture and an amount of water between 0 (excluded) and 5 % by weight of the reaction mixture is maintained.

6. Method according to claim 5, **characterised in that** the reaction is carried out with a weight ratio of methanol reagent feeding the reactor to the methyl formate reagent feeding the reactor of between 0.01 and 100 (included).

7. Method according to any one of claims 1 to 6, **characterised in that** the reaction is carried out in maintaining a partial carbon monoxide pressure greater than or equal to 0.5 . 10⁵ Pa, preferably greater than or equal to 10⁵ Pa.

8. Method according to any one of claims 1 to 7, **characterised in that** the reaction is carried out in maintaining a partial carbon monoxide pressure lower than or equal to 15 . 10⁵ Pa, preferably lower than or equal to 10 . 10⁵ Pa.

9. Method according to any one of claims 1 to 8, **characterised in that** the reaction is carried out in maintaining an amount of water between 0 (excluded) and 2% by weight of the reaction mixture.

10. Method according to any one of claims 1 to 9, **characterised in that** the reaction is carried out in maintaining an amount of halogenated promoter between 0.1 and 20% by weight of the reaction mixture, preferably between 1 and 15% by weight of the reaction mixture.

11. Method according to any one of claims 1 to 10, **characterised in that** the reaction is carried out in the presence of a solvent which is selected from aliphatic carboxylic acids having 2 to 10 carbon atoms.

12. Method according to any one of claims 1 to 11, **characterised in that** the reaction is carried out in the presence of formic acid at a content kept below 15% by weight of the reaction mixture, and preferably below 12% by weight of the reaction mixture.

13. Method according to claim 12, **characterised in that** the formic acid content is kept below 10% by weight of the reaction mixture.

14. Method according to any one of claims 11 to 13, **characterised in that** the amount of free carboxylic acids present in the reaction mixture is greater than 25% by weight of said mixture.

15. Method according to any one of claims 1 to 14, **characterised in that** the reaction is carried out in the presence of a co-solvent which is methyl acetate.

16. Method according to claim 15, **characterised in that** the weight content of the co-solvent is lower than or equal to that of acetic acid.

17. Method according to any one of claims 1 to 16, **characterised in that** the reaction is carried out in the presence of a halogenated promoter selected from iodinated compounds or a precursor of such compounds.
